# EUROPEAN PATENT SPECIFICATION

(11) **EP 2 939 638 B1**
(45) Date of publication and mention of the grant of the patent: **11.04.2018**
(21) Application number: 14166684.2
(22) Date of filing: 30.04.2014
(51) Int. Cl.: A61F 2/40

(54) **A set for connecting a prosthetic assembly of an artificial joint to a bone**
Satz zum Verbinden einer Prothesenanordnung eines künstliches Gelenks mit einem Knochen
Ensemble pour connecter un ensemble prothétique d'une articulation artificielle à un os

(43) Date of publication of application: 04.11.2015
(73) Proprietor: Zimmer GmbH, 8404 Winterthur (CH)
(72) Inventor: Hopkins, Andrew, 8404 Winterthur (CH)
(74) Representative: Manitz Finsterwald Patentanwälte PartmbB

(56) References cited:
- EP-A1- 1 952 788
- WO-A1-2014/035991
- US-A1- 2012 277 880
- US-A1- 2013 261 753

## Description

The present disclosure relates to a set for connecting a prosthetic assembly of an artificial joint to a bone.

E.g. document WO 2007/057054 A discloses a set of the aforementioned kind. Document WO 2014/035991 A1 discloses a set with the features recited in the preamble of claim 1.

The articulation surface of natural joints can be damaged by wear or injuries to such a degree that a prosthetic component has to replace the damaged natural component of the joint. E.g. in cases of shoulder joint arthritis, where the glenoid has been compromised by articular damage, and in particular the rotator cuff is repairable or intact, the current state of the art is to install a cemented glenoid prosthesis. Cemented glenoid prostheses, however, in some cases suffer due to fixation failures generated via tensile loading incurred by humeral head translation across the glenoid articulation surface. Similar or analog situations and problems are encountered in other joints. Therefore, there is a need for an improved concept to connect a prosthetic component of an artificial joint to a bone that allows an easy yet reliable implantation and fixation of said component.

The set according to the present disclosure comprises a prosthetic assembly according to any of claims 1 and 2.

Accordingly, the coupling means of the prosthetic component and the separate bone anchoring means are adapted to achieve a snap-fit connection with each other.

Providing at least one separate bone anchoring means allows fixing the prosthetic component independently from the prosthetic component to the bone which in many cases facilitates the implantation procedure. The prosthetic component, which is in an implanted state in direct contact with the bone via its bone contacting surface, can then be attached to said bone anchoring means. The connection between the prosthetic component and the separate bone anchoring means can - as outlined above - be a snap-fit connection. In particular, the connection is not permanent, i.e. the prosthetic component can be detached from the bone anchoring means, e.g. during revision procedures. The coupling means described above can be realized by simple constructive features. Moreover, said connection in most cases does not require the use of specialized tools during implantation. The coupling means comprises and/or consist of coupling features such that coupling features provided on the prosthetic component and on the anchoring means may be interconnected with each other to connect the prosthetic component to the bone anchoring means.

A simple yet reliable embodiment of the set according to the present disclosure may be limited to the prosthetic component and a single bone anchoring means. In this case an articulation surface of the prosthetic assembly cooperating with other artificial or natural components of the joint is provided on the prosthetic component. Of course, the prosthetic assembly may comprise more elements in addition to the prosthetic component and a single bone anchoring means. It is e.g. conceivable to provide the articulation surface on a separate component that can be mounted to the prosthetic component. Alternatively or additionally, more than one - in particular exactly two - separate bone anchoring means may be provided.

In further embodiments, a coating promoting bone ingrowth may be provided on either one of the bone contacting surface of the prosthetic component or the bone anchoring means. Also, a porous material enabling bone ingrowth may be provided on either one of the bone contacting surface of the prosthetic component or the bone anchoring means. Said porous material may be provided as a coating, or as a material layer forming part of the structure of the prosthetic component or said at least one bone anchoring means.

It is also conceivable to couple the prosthetic component and the at least one bone anchoring means prior to implantation.

In an embodiment of the set according to the present disclosure, one of the coupling means and the bone anchoring means comprises a male coupling feature and the other one of the coupling means and the bone anchoring means comprises a female coupling feature adapted to receive the male coupling feature. The male coupling feature may comprise a bulge or a male taper. For instance, the male coupling feature may comprise a base portion and a head portion, the base portion having a smaller cross-section than the head portion in a plane perpendicular to a longitudinal extension of the base portion. In particular, the head portion comprises a ball head.

The coupling means may comprise a peg that is arranged on and extending from the bone contacting surface, the peg having a proximal end and a distal end, the proximal end arranged fixed to the bone contacting surface. The peg may be attached to or be formed monolithic with the bone contacting surface of the prosthetic component. A coupling feature may be arranged on the peg. In particular each coupling feature of the prosthetic component may be provided on a peg, and/or each peg is furnished with a coupling feature. The number of pegs and the number of coupling features of the prosthetic component may in particular be the same, and each coupling feature may be provided with one single coupling feature. Multiple pegs may be arranged such that their longitudinal axes extend parallel to each other. Further, all pegs may be arranged with their longitudinal axis parallel to each other.

In a further embodiment of the set according to the present disclosure, a female coupling feature provided on either the prosthetic component or the bone anchoring means comprises a cavity or a female taper. A female coupling feature may be formed by a wall surrounding a cavity, said cavity being configured to receive a male coupling feature. Said cavity may be open at a rim of the wall. In one even more specific embodiment the cavity may form an undercut beyond the rim of the wall thus forming a cavity which in particular serves as a female form-fit coupling feature. There may furthermore be provided at least one slot extending axially from the rim of the wall. Said slot will for instance increase the flexibility of the wall which is particularly useful to insert a male form-fit coupling feature across a bottleneck formed at the rim of the wall into the undercut area of the cavity. It will be well appreciated that in certain embodiments the male coupling feature will at least over parts of its surface snugly fit into the undercut area of the female coupling feature.

In yet another embodiment of the set according to the present disclosure, two or more coupling features are provided on the prosthetic component to improve the coupling between the prosthetic component and the bone anchoring means. A number of bone anchoring means may be provided such that the number of coupling features provided by the bone anchoring means corresponds to the number of coupling features provided on the prosthetic component. In one embodiment each bone anchoring means is provided with one coupling feature and consequently the number of bone anchoring means provided will be equal to the number of coupling features provided on the prosthetic component. It is well understood that a coupling feature provided on the prosthetic component and a corresponding coupling feature on the bone anchoring means should be complementary in the sense that the coupling features need to be adapted to fit and lock with each other.

At least two separate bone anchoring means may be provided, wherein each of the coupling means provided on the prosthetic component is associated with one of the bone anchoring means. Said embodiment may be found useful to enhance the distribution of the loads induced by the bone anchoring means. One embodiment of the set comprises exactly two bone anchoring means, each provided with one single coupling feature, and exactly two respective coupling features provided on the prosthetic component.

However, it is possible to choose the number of coupling features freely as needed to ensure a reliable fixation of the prosthetic component to the at least one bone anchoring means. It is even conceivable to provide both male and female coupling features on the prosthetic component and to provide the bone anchoring means with a complementary arrangement of coupling features and/or to provide two or more separate bone anchoring means. For example a peg of the aforementioned kind and/or a bone anchoring means may comprise more than one coupling features.

In a further embodiment of the set according to the present disclosure, the female coupling feature is provided on a bone anchoring means.

In a further embodiment of the set according to the present disclosure, the prosthetic component is made at least partly or entirely from a synthetic material, in particular from plastic such as polyethylene.

The at least one bone anchoring means may comprise a distal bone anchoring section and a proximal head section. The bone anchoring section may be formed as a threaded shank, wherein any one of the core diameter and the outer thread diameter may be cylindrical or tapering. As it will be appreciated any thread form known in orthopedic surgery will be possible, including, but not limited to, continuous, interrupted, self-tapping and double-lead threads. It will furthermore be appreciated that the bone anchoring section may be formed in any other manner suitable to provide adequate bone fixation, such as, but not limited to, press fit in the bone, cementing, or fixation through structures formed on the bone anchoring section, such as for instance a porous surface or barbs. The head section may comprise or be formed as a coupling feature. It will be well understood that a coupling feature provided on a bone anchoring means intended for being coupled with a coupling feature provided on the prosthetic component will be a counterpart coupling feature to that provided on the prosthetic component.

The bone anchoring means will in one embodiment be provided with a socket for a tool enabling inserting the bone anchoring means into the bone. In particular if the bone anchoring section is formed as a thread there may be provided a socket for a screwing tool. In one exemplary embodiment the may for instance be provided a slot as to receive a screwdriver. The head may for instance be formed by a wall, said wall surrounding a cavity forming a female form fit coupling feature wherein the wall comprises an axial slot which serves to enhance the elasticity of the wall and may at the same time serve as a screwdriver socket, as exemplarily lined out above. In another exemplary embodiment there may be provided a ball head as a male coupling feature, said ball head being provided with a hex socket or a cross recess on a proximal face.

The bone anchoring means may be made from any suitable material having appropriate mechanical properties. It will be understood that the bone anchoring means will be made from a biocompatible material, or will at least be furnished with a biocompatible cover or coating. Suitable materials may comprise, but are not limited to, metals such as e.g. stainless steel or titanium. The bone anchoring means, and in particular the bone anchoring section, may be at least partly furnished with a coating promoting bone on-growth or a porous material allowing bone in-growth, such as for instance Zimmer Trabecular Metal™. It is further understood that such coatings or porous material will be most effective when applied to a threaded shank or any other part of the bone anchor intended to effect the anchor-bone connection. The bone anchoring means may comprise, at least in parts of its extent, a solid core surrounded by a sleeve of such porous material, or a tip made of such porous material, analogously to the lag screw disclosed in WO2008/102016.

The present disclosure also relates to a prosthetic component for an artificial joint, in particular for a shoulder prosthesis. The prosthetic component is in particular suited to be used in embodiments of the set described herein. The prosthetic component has an articulation surface and a bone contacting surface contacting the bone in an implanted state of the prosthetic component. The bone contacting surface of the prosthetic component is provided with at least one coupling means. Said coupling means may comprise a male or a female coupling feature, such as a male or a female form-fit or tight-fit coupling feature. The coupling feature may be provided on a peg, said peg being provided on and projecting from the bone contacting surface. For instance, a male form-fit coupling feature may comprise a ball head provided on a distal end of a peg, the peg in turn being provided on and extending from the bone contacting surface. More than one peg and/or more than one coupling feature may be provided.

A method for implanting a set according to any of the embodiments described above
does not form part of the invention and is only described for illustrative purposes hereafter,
the said methods
may comprise placing the at least one bone anchoring means in a bone of a patient and coupling the prosthetic component to the bone anchoring means. In particular, prior to the step of placing the bone anchoring means in the bone of the patient and/or the step of coupling the prosthetic component to the bone anchoring means, the bone is resected to form a component contacting surface adapted to be in contact with the prosthetic component. In particular the mentioned method steps may be carried out in said order.

According to an embodiment of the method, holes for receiving an anchoring section of the bone anchoring means are formed in the bone of the patient, in particular by drilling or milling, prior to placing the bone anchoring means. The holes formed in the bone of the patient may be smaller than an outer dimension of the anchoring section of the bone anchoring means and larger than or essentially equal to a core diameter of the anchoring section. The holes formed in the bone of the patient may be smaller than a core diameter of the anchoring section such as to achieve a compression of bone stock upon placing the bone anchoring means. A countersink may be provided in the patient's bone and the bone anchoring means may be placed such that the anchoring section and the head section are fully received within the bone. The method may comprise selecting areas of relatively denser and/or more stable bone stock, as compared to the overall available bone stock and selecting the location and direction of the insertion of the anchoring means to place the anchoring sections at least partly in said areas.

More areas of applicability of the present invention will become apparent from the detailed description provided hereinafter. It should be understood that the detailed description and specific examples are intended for the purpose of illustration only. The drawings are simplified and schematic. Details not necessary for the understanding of the invention are omitted.

The present disclosure will be explained in more detail and become fully understood from the detailed description and the accompanying drawings.
- Fig. 1: shows a first embodiment of a prosthetic component according to the present disclosure,
- Fig. 2: shows a first embodiment of a bone anchoring means according to the present disclosure,
- Fig. 3a and 3b: show a set comprising the prosthetic component of Fig. 1 and two bone anchoring means according to Fig. 2 in different mounting situations,
- Fig. 4: shows a top view of the bone anchoring means of Fig. 2, and
- Fig. 5: shows second embodiments of a prosthetic component and bone anchoring means according to the present disclosure.

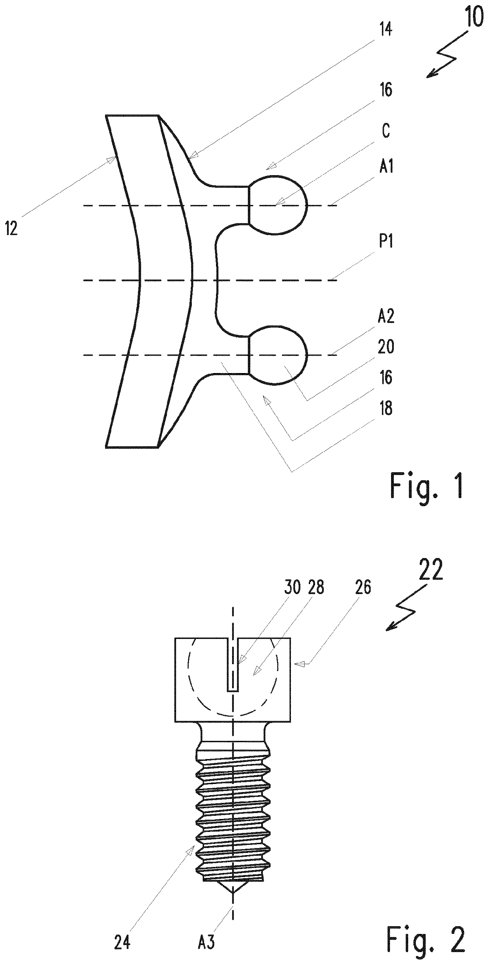

Fig. 1 shows a prosthetic component 10 which is used to replace a damaged natural glenoid surface of a natural shoulder joint. An articular surface 12 of prosthetic component 10 mimics the geometry of the natural glenoid surface. Alternatively, the geometry of said surface may be optimized for cooperation with further prosthetic components of the artificial joint, such as a prosthetic humeral articular surface or an articular surface of a bearing component disposed between prosthetic component 10 and a humeral component of the shoulder prosthesis. The shape of articular surface 12 may be symmetrical or asymmetrical.

Prosthetic component 10 is in an implanted state in direct contact with the surrounding bone. Specifically, a bone contacting surface 14 directly contacts the scapula of the patient in an implanted state. Hence, there is no intermediate component provided which is disposed between the bone surface and prosthetic component 10. The bone surface may be resected to make sure that bone contacting surface 14 of prosthetic component 10 snuggly fits on the bone surface.

It is conceivable to provide a multipartite prosthetic component, e.g. wherein the articular surface and the bone contacting surface are provided on separate but (releasably) joinable elements. Thus, these joinable elements form in a mounted state a multi-part assembly. Mounting of said elements may take place prior to the connection of the element carrying the bone contacting surface to the bone anchoring means or later.

Two pegs 16 extend from bone contacting surface 14. Pegs 16 comprise an essentially cylindrical base portion 18 carrying an essentially spherical ball head 20, said spherical ball head providing a male form fit coupling feature. However, deviations of a spherical shape are conceivable.

Prosthetic component 10 is - as can be seen from Fig. 1 - provided with two pegs 16 disposed symmetrically with respect to a midplane P1, which in the shown embodiment also represents a symmetry plane of surfaces 12, 14. However, it is also possible to provide an asymmetrical design of surfaces 12, 14 and/or an asymmetrical arrangement of pegs 16 on bone contacting surface 14 with respect to midplane P1.

Both pegs 16 extend from bone contacting surface 14 in a direction parallel to plane P1. In other words, each peg 16 defines a longitudinal axis A1, A2, respectively, which is arranged parallel to plane P1. A respective center C of ball heads 20 lies on longitudinal axes A1, A2, respectively.

For specific applications, it might be envisaged to provide pegs 16 having longitudinal axes A1, A2 that are not disposed symmetrically with respect to plane P1 and/or which are not parallel to plane P1. The longitudinal extension of pegs 16 and the geometry of the respective ball heads 20 may be chosen in accordance with the constraints of the specific case and/or the joint geometry or kinematics. It is also not imperative to provide pegs with identical or similar design. To ensure proper installation - especially in case a prosthetic component 10 of asymmetrical design is used - it might be helpful to provide pegs having distinctly different design. These pegs then may be coupled only to bone anchoring means of complementary design so that there is only one way - namely the correct way - of mounting the corresponding prosthetic component to the bone.

E.g. in deviation from the design shown in Fig. 1, prosthetic component 10 may have a first peg 16 that is provided with a female coupling feature and a second peg 16 as depicted in Fig. 1. Further, instead of two pegs 16 only one peg 16 or more than two pegs 16 may be provided.

Fig. 2 shows a bone anchoring means 22 comprising a threaded shank 24 and a head portion 26. Head portion, or distal end, 26 is provided with a cavity 28 (indicated by dashed lines) designed to receive ball head 20 of pegs 16, thus providing a female form-fit coupling feature. Head portion 26 further comprises slots 30 extending parallel to longitudinal axis A3 of bone anchoring means 22. Head portion 26 is designed such that a ball head 20 as illustrated in Fig. 1 can be snapped into said head portion 26.

Exemplarily, two bone anchoring means 22 are mounted in an appropriate way into the resected scapula of a patient. Slots 30 facilitate the use of a suitable screwdriver to screw means 22 in the bone. Then, prosthetic component 10 is snapped onto bone anchoring means 22 through impacting the articular surface 12 in a direction approximately parallel to axes A1, A2 of pegs 16.

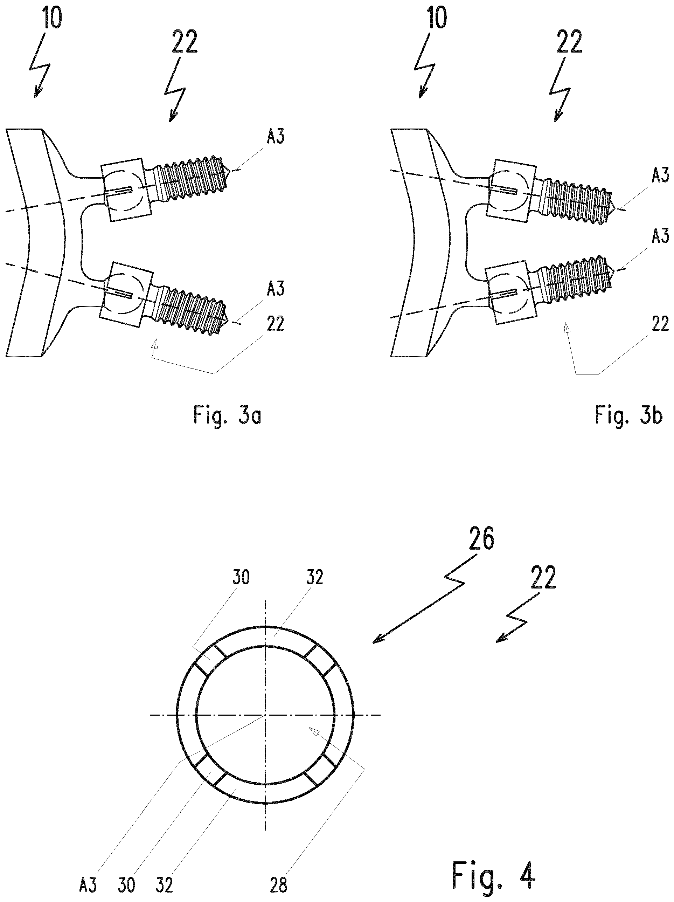

Fig. 3a and 3b each show an example of a set comprising prosthetic component 10 and two bone anchoring means 22 in a mounted state (bone omitted for clarity). In the example shown in Fig. 3a, longitudinal axes A3 of bone anchoring means 22 diverge when seen from bone contacting surface 14. In Fig. 3b, a convergent arrangement of axes A3 is shown.

While in the exemplary embodiments shown in Fig. 3a, 3b the bone anchoring means 22 are arranged with some symmetry with respect to the prosthetic component 10, it will be appreciated that the bone anchoring means 22 need not necessarily to be arranged with any symmetry nor need they be arranged in a common plane. Due to the ball-and-socket joint type connection between the coupling features 20, 28 provided on the prosthetic component 10 and those provided on the bone anchoring means 22, respectively, the bone anchoring means 22 may be arranged in a variety of spatial orientations with respect to prosthetic component 10. In other words, the orientation of the bone anchoring means 22 as implanted into the patient can be chosen relatively freely. The prosthetic component 10 only imparts few geometrical constraints as regards the position and orientation of the implanted bone anchoring means 22. This allows the surgeon to implant the bone anchoring means 22 in regions of the bone where good bone quality can be expected.

It will also be appreciated that male and female coupling features provided on the prosthetic component 10 and a bone anchoring means 22 may be swapped. It will further be appreciated that female as well as male coupling features may be provided on the prosthetic component 10 and the corresponding bone anchoring means 22 then will be chosen to have a complementary coupling feature. The orientation of bone anchors 22 in the bone of the patient may be chosen as required to achieve a reliable fixation. The orientation of bone anchoring means 22 may take the bone quality and other aspects, such as implantation procedures, into account to achieve optimum results. Contrary to conventional concepts, the coupling concept to mount prosthetic component 10 to bone anchoring means 22 comprising in the depicted embodiment a snap-fit connection between ball heads 20 on pegs 16 engaging cavities 28 in head portions 26 of anchors 22 allows a multitude of different bone anchoring means placement geometries, thus giving greater flexibility with one and the same prosthetic component.

Fig. 4 shows a top view of head portion 26 of bone anchoring means 22 revealing that slots 30 not only provide the required flexibility of walls 32 defining cavity 28 necessary to realize a snap-on connection but they can also be used to screw bone anchoring means 22 into the bone of the patient. In other words, slots 30 can be engaged by an appropriate tool, such as a screwdriver, to screw bone anchoring means 22 into the bone.

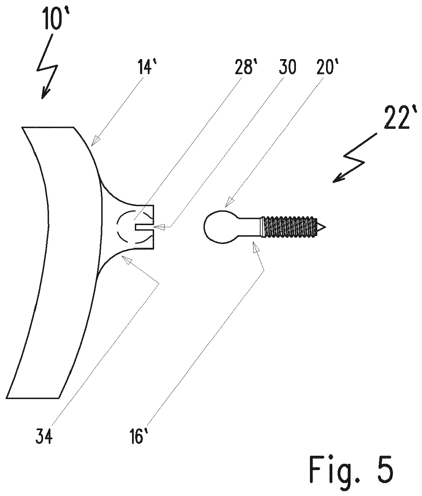

Fig. 5 shows a configuration of a set for connecting a prosthetic assembly of an artificial wherein the prosthetic component 10' of said assembly is provided with a female coupling feature 28' and a single bone anchoring means 22' is provided with the complementary male coupling feature 20'. In other words, proximal end portion 16' of the bone anchoring means 22' comprises ball head 20' providing a male coupling feature and adapted to be received in the female coupling feature 28' provided on the prosthetic component 10'. Female prosthetic component coupling feature 28' - in the embodiment shown in Fig. 5 a cavity - may likewise be provided on a peg 16, analogous to male coupling feature 20 shown in Fig. 1.

It should be understood, that the ball-snap mechanism shown in two alternative variants in Figs. 1 to 5 may be replaced or complemented by other types of connection such as a press-fit connection, e.g. a taper lock connection, and/or other types of a form-fit connection. I.e. embodiments of the set according to the present disclosure are conceivable that are provided with two or more different types of connections. Multiple types of connectors may by be provided at different locations of a prosthetic component.

The connection(s) between a prosthetic component 10, 10' and bone anchoring means 22, 22' may be releasable to allow an easy replacement of the prosthetic component 10, 10'. Bone cement may be additionally used, if necessary. In special cases, prosthetic component 10, 10' may also be cemented directly to the bone.

### Reference numeral list

- 10, 10': prosthetic component
- 12: articular surface
- 14, 14': bone contacting surface
- 16: peg
- 16': proximal end portion
- 18: base portion
- 20, 20': ball head
- 22, 22': bone anchoring means
- 24: threaded shank
- 26: head portion
- 28,28': cavity
- 30: slot
- 32: wall
- 34: mounting portion
- P1: mid plane
- A1, A2, A3: longitudinal axis
- C: center

## Claims

1. A set for connecting a prosthetic assembly of an artificial joint, in particular a glenoidal component of a shoulder prosthesis, to a bone, the set comprising:
- a prosthetic component (10, 10') comprising a bone contacting surface (14, 14') contacting the bone in an implanted state of the prosthetic component and
- separate bone anchoring means (22, 22'),
wherein the bone contacting surface of the prosthetic component is provided with a coupling means (16, 20, 34) adapted to be coupled to the bone anchoring means in a mounted state of the prosthetic component, **characterized in that**
the coupling means and the bone anchoring means are adapted to achieve a snap-fit connection with each other.

2. A set according to claim 1, wherein one of the coupling means and the bone anchoring means comprises a male coupling feature (20, 20') and wherein the other one of the coupling means and the bone anchoring means comprises a female coupling feature (28, 28') adapted to receive the male coupling feature.

3. A set according to claim 2, wherein the male coupling feature (20, 20') comprises a bulge or a male taper.

4. A set according to claims 2 or 3, wherein the coupling means comprises a base portion (16) and a head portion (20, 20'), the base portion having a smaller cross-section than the head portion in a plane perpendicular to a longitudinal extension of the male coupling feature, in particular wherein the head portion comprises a ball head (20, 20').

5. A set according to any of claims 2 to 4, wherein the coupling means comprises a peg (16) that is arranged on and extending from the bone contacting surface (14), the peg having a proximal end and a distal end, the proximal end arranged fixed to the bone contacting surface and wherein a coupling feature (20) is arranged at the distal end of the peg.

6. A set according to any of claims 2 to 5, wherein the female coupling feature comprises a cavity (28, 28') or a female taper.

7. A set according to any of claims 2 to 6, wherein at least two coupling features (20) are provided on the prosthetic component (10).

8. A set according to claim 7, wherein at least two separate bone anchoring means (22, 22') are provided and wherein each of the coupling features (16, 34) provided on the prosthetic component is associated with one of the bone anchoring means.

9. A set according to claims 7 or 8, wherein longitudinal axes (A1, A2) of the at least two male coupling features or of at least two pegs each supporting a coupling feature are arranged in parallel.

10. A set according to any of claims 2 to 9, wherein the bone anchoring means comprises at least one of a male or a female coupling feature (28').

11. A set according to any of claims 2 to 10, wherein the female coupling feature (28, 28') is formed by a cavity surrounded by a wall (32), and wherein said wall is provided with at least one slot (30) extending axially from a rim of the wall defining said female coupling feature.

12. A set according to any of the preceding claims, wherein the prosthetic component (10, 10') is made at least partly or entirely from a synthetic material, in particular from plastic such as polyethylene.

13. A set according to any of the preceding claims, wherein bone anchoring means (22, 22') comprises at least a threaded shank (24) or a bone screw.

## Patentansprüche

1. Satz zur Verbindung einer Prothesenbaugruppe eines künstlichen Gelenks, insbesondere einer Glenoidkomponente einer Schulterprothese mit einem Knochen, wobei der Satz umfasst:
- eine Prothesenkomponente (10, 10'), die eine Knochenkontaktfläche (14, 14') umfasst, die mit dem Knochen in einem implantierten Zustand der Prothesenkomponente in Kontakt steht, und
- ein separates Knochenverankerungsmittel (22, 22'),
wobei die Knochenkontaktfläche der Prothesenkomponente mit einem Kopplungsmittel (16, 20, 34) versehen ist, das zur Kopplung mit dem Knochenverankerungsmittel in einem montierten Zustand der Prothesenkomponente angepasst ist,
**dadurch gekennzeichnet, dass**
das Kopplungsmittel und das Knochenverankerungsmittel derart ausgebildet sind, dass sie miteinander eine Schnapppassungsverbindung erzielen.

2. Satz nach Anspruch 1, wobei eines von dem Kopplungsmittel und dem Knochenverankerungsmittel ein Stiftkopplungsmerkmal (20, 20') umfasst, und wobei das andere des Kopplungsmittels und des Knochenverankerungsmittels ein Buchsenkopplungsmerkmal (28, 28') umfasst, das zur Aufnahme des Stiftkopplungsmittels angepasst ist.

3. Satz nach Anspruch 2, wobei das Stiftkopplungsmerkmal (20, 20') einen Wulst oder einen Stiftkonus umfasst.

4. Satz nach einem der Ansprüche 2 oder 3, wobei das Kopplungsmittel einen Basisabschnitt (16) und einen Kopfabschnitt (20, 20') umfasst, wobei der Basisabschnitt einen kleineren Querschnitt als der Kopfabschnitt in einer Ebene rechtwinklig zu einer Längserstreckung des Stiftkopplungsmerkmals aufweist, wobei insbesondere der Kopfabschnitt einen Kugelkopf (20, 20') umfasst.

5. Satz nach einem der Ansprüche 2 bis 4, wobei das Kopplungsmittel einen Zapfen (16) umfasst, der an der Knochenkontaktfläche (14) angeordnet ist und sich von dieser erstreckt, wobei der Zapfen ein proximales Ende und ein distales Ende aufweist, wobei das proximale Ende an der Knochenkontaktfläche fixiert angeordnet ist, und wobei ein Kopplungsmerkmal (20) an dem distalen Ende des Zapfens angeordnet ist.

6. Satz nach einem der Ansprüche 2 bis 5, wobei das Buchsenkopplungsmerkmal einen Hohlraum (28, 28') oder einen Buchsenkonus umfasst.

7. Satz nach einem der Ansprüche 2 bis 6, wobei zumindest zwei Kopplungsmerkmale (20) an der Prothesenkomponente (10) vorgesehen sind.

8. Satz nach Anspruch 7, wobei zumindest zwei separate Knochenverankerungsmittel (22, 22') vorgesehen sind und wobei jedes der Kopplungsmerkmale (16, 34), die an der Prothesenkomponente vorgesehen sind, einem des Knochenverankerungsmittels zugeordnet sind.

9. Satz nach einem der Ansprüche 7 oder 8, wobei Längsachsen (A1, A2) der zumindest zwei Stiftkopplungsmerkmale oder zumindest zwei Zapfen, die jeweils ein Kopplungsmerkmal tragen, parallel angeordnet sind.

10. Satz nach einem der Ansprüche 2 bis 9, wobei das Knochenverankerungsmittel zumindest eines von einem Stift- oder einem Buchsenkopplungsmerkmal (28') umfasst.

11. Satz nach einem der Ansprüche 2 bis 10, wobei das Buchsenkopplungsmerkmal (28, 28') von einem durch eine Wand (32) umgebenen Hohlraum gebildet ist, und wobei die Wand mit zumindest einem Schlitz (30) versehen ist, der sich axial von einer Einfassung der das Buchsenkopplungsmerkmal definierenden Wand erstreckt.

12. Satz nach einem der vorhergehenden Ansprüche, wobei die Prothesenkomponente (10, 10') zumindest teilweise oder vollständig aus einem synthetischen Material, insbesondere aus Kunststoff, wie Polyethylen, hergestellt ist.

13. Satz nach einem der vorhergehenden Ansprüche, wobei das Knochenverankerungsmittel (22, 22') zumindest einen Gewindeschaft (24) oder eine Knochenschraube umfasst.

## Revendications

1. Ensemble pour connecter un assemblage prothétique d'une articulation artificielle, en particulier un composant glénoïdien d'une prothèse d'épaule, à un os, l'ensemble comprenant :
- un composant prothétique (10, 10') comprenant une surface de contact sur os (14, 14') en contact avec l'os dans un état implanté du composant prothétique, et
- des moyens d'ancrage dans l'os séparés (22, 22'),
dans lequel la surface de contact sur os du composant prothétique est dotée d'un moyen de couplage (16, 20, 34) adapté pour être couplé au moyen d'ancrage dans l'os dans un état monté du composant prothétique,
**caractérisé en ce que**
le moyen de couplage et le moyen d'ancrage pour os sont adaptés à réaliser une connexion à encliquetage l'un avec l'autre.

2. Ensemble selon la revendication 1, dans lequel un moyen parmi le moyen de couplage et le moyen d'ancrage pour os comprend une caractéristique de couplage mâle (20, 20') et dans lequel l'autre moyen parmi le moyen de couplage et le moyen d'ancrage pour os comprend une caractéristique de couplage femelle (28, 28') adaptée pour recevoir la caractéristique de couplage mâle.

3. Ensemble selon la revendication 2, dans lequel la caractéristique de couplage mâle (20, 20') comprend un bombement ou une partie effilée mâle.

4. Ensemble selon la revendication 2 ou 3, dans lequel le moyen de couplage comprend une portion de base (16) et une portion de tête (20, 20'), la portion de base ayant une section transversale plus petite que la portion de tête dans un plan perpendiculaire à une extension longitudinale de la caractéristique de couplage mâle, en particulier dans lequel la portion de tête comprend une tête sphérique (20, 20').

5. Ensemble selon l'une quelconque des revendications 2 à 4, dans lequel le moyen de couplage comprend un téton (16) qui est agencé sur et qui s'étend depuis la surface de contact sur os (14), le téton ayant une extrémité proximale et une extrémité distale, l'extrémité proximale étant agencée fixée à la surface de contact sur os, et dans lequel une caractéristique de couplage (20) est agencée à l'extrémité distale du téton.

6. Ensemble selon l'une quelconque des revendications 2 à 5, dans lequel la caractéristique de couplage femelle comprend une cavité (28, 28') ou une partie effilée femelle.

7. Ensemble selon l'une quelconque des revendications 2 à 6, dans lequel au moins deux caractéristiques de couplage (20) sont prévues sur le composant prothétique (10).

8. Ensemble selon la revendication 7, dans lequel au moins deux moyens d'ancrage dans l'os séparés (22, 22') sont prévus, et dans lequel chacune des caractéristiques de couplage (16, 34) prévues sur le composant prothétique est associée avec l'un des moyens d'ancrage dans l'os.

9. Ensemble selon les revendications 7 ou 8, dans lequel des axes longitudinaux (A1, A2) desdites au moins deux caractéristiques de couplage mâles ou d'au moins deux tétons supportant chacun une caractéristique de couplage sont agencés en parallèle.

10. Ensemble selon l'une quelconque des revendications 2 à 9, dans lequel le moyen d'ancrage dans l'os comprend au moins une caractéristique de couplage mâle ou femelle (28').

11. Ensemble selon l'une quelconque des revendications 2 à 10, dans lequel la caractéristique de couplage femelle (28, 28') est formée par une cavité entourée par une paroi (32), et dans lequel ladite paroi est pourvue d'au moins une fente (30) s'étendant axialement depuis une bordure de la paroi définissant ladite caractéristique de couplage femelle.

12. Ensemble selon l'une quelconque des revendications précédentes, dans lequel le composant prothétique (10, 10') est réalisé au moins partiellement ou entièrement à partir d'une matière synthétique, en particulier à partir d'une matière plastique telle que du polyéthylène.

13. Ensemble selon l'une quelconque des revendications précédentes, dans lequel le moyen d'ancrage dans l'os (22, 22') comprend au moins une tige filetée (24) ou une vis à os.
